# EUROPEAN PATENT APPLICATION

(11) **EP 1 283 032 A1**
(43) Date of publication of application: **12.02.2003**
(21) Application number: 02255595.7
(22) Date of filing: 09.08.2002
(51) Int. Cl.: A61K 7/48, A61P 17/00

(54) **Skin care composition that changes color upon drying**

(30) Priority: 10.08.2001 US 928110
(71) Applicant: JOHNSON & JOHNSON CONSUMER COMPANIES, INC., Skillman, NJ 08558 (US)
(72) Inventor: Dole, Victoria F., Whitehouse Station, NJ 08889 (US); Robertson, Katherine, Plainsboro, NJ 08536 (US)
(74) Representative: Mercer, Christopher Paul

(57) **Abstract**

A composition for forming a cosmetic mask including: at least one colorant; and silica, wherein when the composition is combined with water, the amount of the colorant and the silica is effective to provide a color change upon drying is disclosed. The composition may be useful as a skin care formulation, such as for a facial mask, a cleanser, and the like.

## Description

### Background of the Invention

### 1. Field of the Invention

This invention relates to a skin care composition that has many uses, including a cleansing facial mask. The composition may be a paste, lotion, gel, or cream that is applied to the skin. Upon drying, the composition changes color, indicating that it is time to be rinsed off.

### 2. Description of the Prior Art

Facial masks are formulations that typically are used to cleanse, exfoliate, and moisturize the face. They are referred to as masks because the formulations are applied to the face and left on for some period of time. The eyes, mouth, and nostrils are avoided when applying the formulations, therefore the formulation appears to form a mask. Some formulations are applied wet and rinsed off while still wet. In this case, the user keeps track of the time elapsed since applying the mask, then washes the mask off after the appropriate time has elapsed. Other formulations are applied wet, allowed to dry, then rinsed off or peeled off.

For formulations that are dried prior to removal, the formulation dries over a period of time that will vary depending on the components, however generally does not exceed twenty minutes. Depending on the formulation, the mask may be peeled off or rinsed off. Peel off masks typically contain a polymer that forms a film on the skin. Typical polymers include polyvinyl alcohol, polvinyl pyrrolidone, and other water-soluble polymers.

It is relatively easy to apply a facial mask to the face. The formulation is typically placed on the fingers and transferred to the face. Most facial mask users apply the formulation to the entire surface of the face, except the eyes, mouth, and nostrils. With the use of a mirror, it is easy to see the areas of the face that are not covered with the formulation, and apply more as needed.

It is sometimes difficult to determine when it is time to remove a dry facial mask. The mask should be left on long enough to provide the cleansing, exfoliating, and moisturizing properties it is meant to deliver. Facial mask formulations are typically prepared such that the proper leave on time for the desired benefits is equal to the drying time of the formulation. Therefore, typical facial masks should be removed when the formulation has completely dried.

With many facial mask formulations, it is difficult to tell when the formulation has completely dried. Although one can determine the time to dry experimentally and then time each facial mask application, this approach may be inconvenient for many. The inconvenience is due to the fact that facial masks typically are applied at night while preparing for bed, frequently at the bathroom sink. Many people do not wear watches while preparing for bed and do not have clocks in their bathrooms. Therefore, it may be inconvenient to check the time that the facial mask was applied and the time elapsed since applying the facial mask. Therefore, there is a need for a facial mask that provides a change in appearance that indicates it is dry.

The self-heating mask, produced by Biore contains an aquamarine colorant. The formulation is off white with blue specks prior to application on the face. As the formulation is applied to the face, the color becomes more evenly distributed and the formulation appears aquamarine in color. The mask is rinsed off when the aquamarine color is present and the mask is still wet. The color does not change upon drying. Despite the disclosure of the prior art, there is a continuing need for a facial mask that provides a change in appearance that indicates it is dry.

### Summary of the Invention

The present invention provides a composition for forming a cosmetic mask including: at least one colorant; and silica, wherein when the composition is combined with water, the amount of the colorant and the silica is effective to provide a color change upon drying.

### Detailed Description of Preferred Embodiments

The compositions of the invention may be supplied as powders, to be mixed with water and applied to the skin. Alternatively, the compositions of the invention may be supplied in liquid formulations, such as pastes, creams, lotions, gels and the like.

At least one colorant is added to the compositions of this invention in an effective amount to provide the color change that indicates the composition has dried sufficiently. Suitable colorants include, but are not limited to, synthetically derived colorants such as FD&C Blue #1, FD&C Green #3, FD&C Blue #1 Lake, FD&C Blue #2 Lake, FD&C Red #40 Lake, Erythrosin Lake, Amaranth Lake, Ponceau 4R Lake, Carmoisosine Lake, Carmine Lake, natural dyes such as, but not limited to, anatto and the like, and colorants generated by converting a naturally derived dye to an aluminum or calcium based salt. The amount of colorant typically ranges from about 0.0001 to about 0.1, preferably from about 0.0001 to about 0.01 percent by weight, based on the total weight of the composition.

Silica is added to the compositions of this invention in an effective amount in order to change the color of the composition upon drying. Silica, when wet is translucent therefore the color of the colorant dominates the wet composition. Upon drying, silica is opaque and appears white. Therefore, when the composition is dry, the color changes from the color of the colorant to either a lighter shade of the color of the colorant or white. Silica may be used in hydrated or anhydrous form. It is preferable to utilize amorphous forms of silica. Suitable amorphous forms of silica include, but are not limited to, fumed silica, hydrated silica, silica gels, precipitated silica, and molecular sieves. As described above, the silica is added to the compositions of this invention in an effective amount in order to change the color of the composition upon drying. The amount of silica may range from about 1% to about 99.99%, preferably from about 5% to about 40%, more preferably from about 10% to about 30% by weight of the total composition.

For liquid formulations, water is the preferred vehicle for the compositions of the invention. The amount of water utilized will depend on the amounts of the required components and any optional components in the formulation. Generally, the amount of water may range from about 40 to about 90, preferably from about 45 to about 75 percent by weight, based on the total weight of the composition.

Other suitable vehicles include water miscible liquids including, but not limited to, alcohols, such as, but not limited to, ethyl alcohol, propyl alcohol, and butyl alcohol; glycols, such as, but not limited to, ethylene glycol, dipropylene glycol, and butylene glycol, and mixtures of water miscible liquids and water. The amount of water miscible liquid may range from about 1 to about 20 percent by weight, based on the total weight of the composition.

An emollient may be included in the compositions of the invention. Emollients function by their ability to remain on the skin surface or in the stratum corneum to act as lubricants, to reduce flaking, and to improve the skin appearance. Typical emollients include fatty esters, fatty alcohols, mineral oil, polyether siloxane copolymers and the like. Examples of suitable emollients include, but are not limited to, polypropylene glycol ("PPG")-15 stearyl ether, PPG-10 cetyl ether, steareth-10, oleth-8, PPG-4 lauryl ether, C₁₂ - C₁₅ alkyl benzoates, vitamin E acetate, PEG-7 glyceryl cocoate, lanolin, glycerine, and combinations thereof. Glycerine, vitamin E acetate, PEG-7 glyceryl cocoate, C₁₂ - C₁₅ alkyl benzoates, and combinations thereof are preferred. The emollient can be present in an amount from about 0.1 to about 30, preferably from about 1 to about 20, more preferably from about 5 to about 15 percent by weight, based on the total weight of the composition.

The compositions of the invention may also include at least one cleansing agent. Suitable cleansing agents include, but are not limited to, nonionic surfactants, anionic surfactants, cationic surfactants, amphoteric surfactants, and mixtures thereof.

Types of nonionic surfactants that are suitable for use in this invention include, but are not limited to, the fatty alcohol acid or amide ethoxylates, monoglyceride ethoxylates, sorbitan ester ethoxylates and alkyl polyglycosides. These nonionic surfactants can be employed in the composition of the present invention in an amount, based upon the total weight of the composition, from about 0.1% to about 10%, e.g. from about 0.5% to about 8% and from about 1% to about 5%.

Classes of anionic surfactants useful in this invention include, but are not limited to, the alkyl sulfates, alkyl ether sulfates, sulfosuccinates, isethionates, acyl amides, alkyl ether carboxylates and alkyl phosphates, wherein the alkyl group has from about 6 carbon atoms to about 30 carbon atoms, with about 10 to about 14 carbon atoms being preferred. These anionic surfactants can be employed in the composition of the present invention in an amount, based upon the total weight of the composition, from about 0.1% to about 10%, e.g. from about 0.5% to about 8% and from about 1% to about 5%.

Classes of cationic surfactants that are suitable for use in this invention include, but are not limited to, alkyl quaternaries (mono, di, or tri), benzyl quaternaries, ester quaternaries, ethoxylated quaternaries, alkyl amines, and mixtures thereof, wherein the alkyl group has from about 6 carbon atoms to about 30 carbon atoms, with about 8 to about 22 carbon atoms being preferred. These cationic surfactants can be employed in composition of the present invention in an amount, based upon the total weight of the composition, from about 0.1% to about 10%, preferably from about 0.5% to about 8% and more preferably from about 1% to about 5%.

Classes of amphoteric surfactants that are suitable for use in this invention include, but are not limited to, alkylimino-diproprionates, alkylamphoglycinates (mono or di), alkylamphoproprionates (mono or di), alkylamphoacetates (mono or di), N-alkyl β-aminoproprionic acids, alkylpolyamino carboxylates, and phosphorylated imidazolines. These amphoteric surfactants can be employed in composition of the present invention in an amount, based upon the total weight of the composition, from about 0.1% to about 10%, e.g. from about 0.5% to about 8% and from about 1% to about 5%.

Types of betaines that are suitable for use in this invention include, but are not limited to, alkyl betaines, alkylamido betaines, alkyl sultaines and alkylamido sultaines, wherein the alkyl group has from about 6 carbon atoms to about 30 carbon atoms, with about 10 to about 14 carbon atoms being preferred. These betaine surfactants can be employed in the cleansing composition of the present invention in an amount, based upon the total weight of the composition, from about 0.1% to about 10%, e.g. from about 0.5% to about 8% and from about 1% to about 5%.

Opacifying agents may be added to the compositions of the invention. Suitable opacifying agents include, but are not limited to talc, titanium dioxide, inorganic materials, nylon 6, nylon 6,6, nylon 11, nylon 12, calcium carbonate and other minerals, and latex emulsions known in the cosmetics art. The amount of opacifying agent may range from about 0.5% to about 15%, based on the total weight of the composition.

Clays may also be added to the compositions of the invention to enhance drying. Suitable clays include, but are not limited to, alumina, bentonite, diatomaceous earth, kaolin, magnesium aluminum silicate, montmorillonite, sodium lithium magnesium silicate, zeolite, and the like. The amount of clay added is effective to enhance drying, and typically ranges from about 0.1% to about 15% by weight of the total composition.

The compositions of the invention may include anti-acne agents. Suitable anti-acne active ingredients include, but are not limited to, salicylic acid, sulfur, lactic acid, glycolic acid, pyruvic acid, urea, resorcinol, N-acetylcysteine, retinoic acid, benzoyl peroxide, octopirox, triclosan, azelaic acid, phenoxyethanol, phenoxypropanol, flavinoids, derivatives thereof, and combinations thereof. Salicylic acid and benzoyl peroxide are preferred. Salicylic acid is most preferred. The amount of anti-acne active ingredient in the composition of the invention may range from about 0.1 to about 5, preferably from about 0.5 to about 2 percent by weight, based on the total weight of the composition.

The compositions of the invention may also include anti-aging agents. Examples of suitable anti- aging, i.e. wrinkles, fine lines, and other manifestations of photodamage, agents include, but are not limited to, inorganic sunscreens such as titanium dioxide and zinc oxide; organic sunscreens such as octyl-methyl cinnamates and derivatives thereof; retinoids; vitamins such as vitamin E, vitamin A, vitamin C, vitamin B, and derivatives thereof such as vitamin E acetate, vitamin C palmitate, and the like; antioxidants including beta carotene, alpha hydroxy acid such as glycolic acid, citric acid, lactic acid, malic acid, mandelic acid, ascorbic acid, alpha-hydroxybutyric acid, alpha-hydroxyisobutyric acid, alpha-hydroxyisocaproic acid, atrrolactic acid, alpha-hydroxyisovaleric acid, ethyl pyruvate, galacturonic acid, glucopehtonic acid, glucopheptono 1,4-lactone, gluconic acid, gluconolactone, glucuronic acid, glucurronolactone, glycolic acid, isopropyl pyruvate, methyl pyruvate, mucic acid, pyruvia acid, saccharic acid, saccaric acid 1,4-lactone, tartaric acid, and tartronic acid; beta hydroxy acids such as beta-hydroxybutyric acid, beta-phenyl-lactic acid, beta-phenylpyruvic acid; botanical extracts such as green tea, soy, milk thistle, algae, aloe, angelica, bitter orange, coffee, goldthread, grapefruit, hoellen, honeysuckle, Job's tears, lithospermum, mulberry, peony, puerarua, nice, safflower, and mixtures thereof.

Preferred anti-aging agents include retinoids, anti-oxidants, alpha-hydroxy acids and beta-hydroxy acid with retinol and tretinoin being most preferred.

Anti-irritant agents may also be useful in the compositions of the invention. Examples of suitable anti-irritant agents include, but are not limited to, colloidal oatmeal, oat extract, agents known for reducing the symptoms of diaper rash such as dimethicone, white petrolatum, zinc oxide, and mixtures thereof and the like.

The compositions of the present invention may include anti-bacterial agents. Suitable anti-bacterial agents include, but are not limited to, ethyl alcohol, Triclosan, benzalkonium chloride, phenoxyethanol, and combinations thereof. The effective amounts of such anti-bacterial agents are well known in the art.

A solubilizer may be included in the compositions of the invention to stabilize the composition so that insoluble ingredients do not precipitate out of solution under normal storage conditions. Suitable solubilizers include, but are not limited to, propylene glycol, butylene glycol, hexylene glycol, polyethylene glycol ("PEG"), polysorbate-20, polysorbate-40, isoceteth-15, isoceteth-20, isoceteth-30, sorbeth-20, sorbeth-40, PEG-40 castor oil, polypropylene glycol-5 ceteth 20, and combinations thereof. As indicated above, the amount of solubilizer added is effective to stabilize the composition so that insoluble ingredients do not precipitate out of solution under normal storage conditions. The amount of solubilizer typically ranges from about 1 to about 20 percent by weight, based on the total weight of the composition.

Thickeners may be added to the compositions of the invention to adjust the viscosity of the composition. Suitable thickeners include, but are not limited to, cellulosic thickeners such as carboxymethyl cellulose and hydroxyethyl cellulose; gum thickeners such as guar gum and xanthan gum, polyethylene glycol 150 distearate, polyethylene glycol 150 pentaerythrityl tetrastearate, and latex thickeners. The amount of thickener may range from about 0.05% to about 5%, based on the total weight of the composition. In the liquid state, the compositions of the invention have a viscosity ranging from about 20,000 cps to about 750,000 cps, preferably from about 100,000 cps to about 500,000 cps.

The compositions of the invention optionally include humectants, UV absorbers, pH adjusting agents, skin soothers, preservatives, conditioning agents, and fragrances.

A humectant may be added to the compositions of the invention in an amount effective to increase moisturization, reduce scaling, stimulate removal of built-up scale, and improve skin feel. Polyhydric alcohols can be utilized as humectants in the compositions of the invention. Suitable polyhydric alcohols include, but are not limited to, glycerol (also known as glycerin), polyalkylene glycols, alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-dibutylene glycol, 1,2,6,-hexanetriol, ethoxylated glycerol, propoxylated glycerol and mixtures thereof. Glycerin and propylene glycol are preferred. When utilized, the humectant is present in an amount from about 0.1 to about 40, preferably from about 1 to about 20, more preferably from about 5 to about 15 percent by weight, based on the total weight of the composition.

Skin soothers and protectants may be added to the compositions of the invention. Suitable skin soothers and protectants include, but are not limited to, panthenol, bisabolol, allantoin, dimethicone, and combinations thereof. When utilized, the skin soother or protectant is present in an amount from about 0.01 to about 5, preferably from about 0.1 to about 2 percent by weight, based on the total weight of the composition.

Conditioning agents may also be added to the compositions of the invention. Suitable conditioning agents include, but are not limited to, dimethicone propyl PG-betaine, dimethicone copolyols, cyclomethicone, polyquaternium-10, and combinations thereof. When utilized, the conditioning agent is present in an amount from about 0.01 to about 5, preferably from about 0.1 to about 3 percent by weight, based on the total weight of the composition.

The pH of the compositions of the invention is from about 3 to about 10, preferably from about 3 to about 8. The pH may be adjusted through the use of pH adjusters, such as, but not limited to sodium citrate. The amount of pH adjuster utilized will depend on the initial pH of the composition and the volume of composition to be adjusted. Generally, the amount of pH adjuster utilized may range from about 0.1 to about 1, preferably form about 0.2 to about 0.4 percent by weight, based on the total weight of the composition.

UV absorbers may be added to the compositions of the invention. Suitable UV absorbers include, but are not limited to, benzophenone and its derivatives, cinnamic acid and its derivatives, azoles, imidazoles and the like. When utilized, the amount of UV absorber ranges from about 0.001 to about 0.01 percent by weight, based on the total weight of the composition.

Botanicals, such as aloe barbadensis extract, chamomile extract, thyme extract, rosemary extract, bitter orange, colts foot, sage, myrrh, and the like may also be useful in the compositions of the invention. When utilized, botanicals are present at from about 0.01 to about 20, preferably from about 0.1 to about 10 percent by weight, based on the total weight of the composition.

Sensates, such as menthyl lactate, menthol, camphor, peppermint, eucalyptus oil, menthoxypropanediol, and the like may also be useful in the compositions of the invention. When utilized, sensates are present at from about 0.01 to about 3, preferably from about 0.05 to about 1 percent by weight, based on the total weight of the composition.

Preservatives are typically added to compositions to inhibit the growth of microbial organisms. Suitable preservatives to be added to the compositions of the invention include benzoic acid, ethylenediamine tetracetic acid, phenoxyethanol, Quatemium-15, available commercially as "Dowicil 200" from the Dow Chemical Corporation of Midland, Michigan, and combinations thereof. Benzoic acid and phenoxyethanol are preferred. When utilized, the preservative is present in an amount from about 0.01 to about 5, preferably from about 0.05 to about 2 percent by weight, based on the total weight of the composition.

Any fragrance may be added to the compositions of the invention for aesthetic purposes. Suitable fragrances include, but are not limited to, eucalyptus oil, camphor synthetic, peppermint oil, clove oil, lavendar, chamomile and the like. When utilized, fragrances are present in an amount from about 0.05 to about 0.5, preferably from about 0.1 to about 0.3 percent by weight, based on the total weight of the composition.

The compositions of the invention are useful for lightening, refreshing, protecting, firming, slimming, cleansing, exfoliating, and moisturizing the skin, as well as for relieving itching, reducing wrinkles, and providing antibacterial protection. The compositions may be supplied as a powder to be mixed with water and applied to the skin, or as a liquid, such as a cream, a paste, a gel, or a lotion. The compositions of the invention may be mixed with commercially available cosmetic products such as a lotion or a cream to provide further benefits to the consumer. The compositions of the invention may be applied to the skin utilizing fingers, cotton swabs, cloths, and the like.

In one embodiment, the composition is applied to the face to create a mask. On application, and while wet, the composition will be the color of the colorant. After drying, the mask will change to a lighter shade of the color of the colorant or to white. The consumer then rinses the mask off. Preferably, the mask composition may be used once or twice a week. The mask composition preferably contains silica, at least one colorant, water, at least one cleansing agent, at least one emollient, and at least one humectant.

In another embodiment, the composition of the invention is applied to other areas of the body, such as the arms and legs. On application, and while wet, the composition will be the color of the colorant. After drying, the composition will change to a lighter shade of the color of the colorant or to white. The consumer then rinses the composition off. Preferably, the composition may be used once or twice a week.

If a peel off mask is desired, the compositions of the invention may include a polymer that forms a film on the skin. Typical polymers include polyvinyl alcohol, polvinyl pyrrolidone, and other water-soluble polymers. The amount of polymer may range from about 1 to about 20 percent by weight, based on the total weight of the composition.

The compositions of the invention may be prepared by means known in the art. For example, the compositions may be prepared by mixing the ingredients described above in a mix tank with commercially available mixing equipment.

The invention also relates to a method of providing a benefit selected from lightening, refreshing, protecting, firming, slimming, cleansing, exfoliating, moisturizing, relieving itching, reducing wrinkles, and providing antibacterial protection to the skin of a human. The method includes applying a composition of the invention to the skin, allowing the composition to dry, and rinsing the composition off.

Examples are set forth below to further illustrate the nature of the invention and the manner of carrying it out. However, the invention should not be considered as being limited to the details thereof.

### Example 1 - Preparation Of A Facial Mask Composition

Skin care compositions were prepared as described below. All weights are in percent by weight based on the total weight of the final composition.

Skin Care Composition A) Phase A included 52.55% deionized water, 0.2% Versene® NA disodium EDTA (Dow Chemical), 0.7% of a 20% solution of sodium citrate (Haarman & Reimer) in water, 1.5% Glycerox® 767 PEG-6 caprylic/capric glycerides (Croda), 6% propylene glycol (Arco Chemical), 1% Abil® B 9950 dimethicone propyl PG-betaine (Goldschmidt), 1% titanium dioxide (Kronos), 17% Syloid® 244FP hydrated silica (Grace Davison), 2% Tegobetaine® L7 cocamidopropyl betaine (Goldschmidt), and 0.8% of a solution of FD&C Blue #1 (Whittaker, Clark & Daniels) in water.

Phase B included 5% Glycerine 916 (Henkel), and 0.5% Xantural® 180 xanthan gum (cpKelco).

Phase C included 2.5% Arlasolve® 200 isoceteth-20 (Uniqema), 0.5% salicylic acid (NIPA), 0.25% Frescolate® menthyl lactate (Haarman & Reimer), 7.5% Finsolv® TN C₁₂-C₁₅ alkyl benzoate (Finetex), 0.7% Phenoxetol® phenoxyethanol (NIPA), and 0.3% fragrance.

The water and EDTA were placed in a beaker and mixed until the EDTA dissolved. The sodium citrate was added and mixed until it dissolved.

Part B (the glycerine and xanthan gum) were combined in a separate beaker and mixed, then added to the water/EDTA/sodium citrate solution (part A).

The following ingredients were added one at a time and mixed until dissolved: Glycerox 767, propylene glycol, and Abil B 9950.

In a separate beaker, Part C was prepared by mixing Arlasolv 200 and Finsolv TN, then adding the following ingredients one at a time and mixing until uniform: salicylic acid, Frescolate, Phenoxetol, and fragrance.

Part C was added to the solution and mixed until uniform. Titanium dioxide was added and mixed until uniform. The mixture was transferred to a Kitchenaid mixer. The silica was added and mixed until uniform. Tegobetaine was added and mixed until uniform, then the dye solution was added and mixed until uniform. The composition formed a light blue, opaque, viscous cream.

Following the same procedure as Example A, the following compositions were prepared:

| Skin Care Composition B) | |
|---|---|
| Component | Amount (% by weight) |
| Water | 75.8 |
| Disodium EDTA | 0.2 |
| Xanthan gum | 0.3 |
| Aerosil® 200 silica | 9 |
| PPG-15 stearyl ether | 5 |
| Polysorbate 60 | 2.5 |
| Ethoxydiglycol | 5 |
| Methoxypropanediol | 0.25 |
| Phenoxetol | 1 |
| Cocamidopropyl betaine | 0.4 |
| Fragrance | 0.3 |
| FD&C Blue #1 (0.1% solution) | 0.25 |

The composition formed a light blue, opaque, viscous cream.

| Skin Care Composition C) | |
|---|---|
| Component | Amount (% by weight) |
| Water | 74.75 |
| Disodium EDTA | 0.2 |
| Xanthan gum | 0.3 |
| Aerosil® 200 silica | 8 |
| Syloid® 244 FP hydrated silica | 1 |
| Glycerine | 1 |
| PPG-15 stearyl ether | 5 |
| Polysorbate 60 | 2.5 |
| Ethoxydiglycol | 5 |
| Methoxypropanediol | 0.20 |
| Phenoxetol | 1 |
| Cocamidopropyl betaine | 0.5 |
| Fragrance | 0.3 |
| FD&C Blue #1 (0.1% solution) | 0.25 |

The composition formed a light blue, opaque, viscous cream.

| Skin Care Composition D) | |
|---|---|
| Component | Amount (% by weight) |
| Water | 72.55 |
| Disodium EDTA | 0.2 |
| Xanthan gum | 1 |
| PEG-6 Caprylic/capric glycerides | 1 |
| Glycerine | 1 |
| Syloid® 244FP hydrated silica | 9 |
| PPG-15 stearyl ether | 5 |
| Polysorbate 60 | 2.5 |
| Hexylene glycol | 5 |
| Menthyl lactate | 0.2 |
| Phenoxetol | 1 |
| Titanium dioxide and octododecyl neopentanoate | 1 |
| Fragrance | 0.3 |
| FD&C Blue #1 (0.1% solution) | 0.25 |

The composition formed a light blue, opaque gel. The viscosity was 23,850 cps as measured on a Brookfield LVT viscometer using spindle #4 at 12 rpm.

| Skin Care Composition E) | |
|---|---|
| Component | Amount (% by weight) |
| Water | 72.55 |
| Disodium EDTA | 0.2 |
| Xanthan gum | 1 |
| PEG-6 Caprylic/capric glycerides | 1.5 |
| Glycerine | 1 |
| Syloid® 244FP hydrated silica | 9 |
| PPG-15 stearyl ether | 5 |
| Polysorbate 60 | 2.5 |
| Hexylene glycol | 5 |
| Menthyl lactate | 0.2 |
| Phenoxetol | 1 |
| Titanium dioxide | 0.5 |
| Fragrance | 0.3 |
| FD&C Blue #1 (0.1% solution) | 0.25 |

The composition formed a pale blue, opaque gel. The viscosity was 20,050 cps as measured on a Brookfield LVT viscometer using spindle #4 at 12 rpm.

| Skin Care Composition F) | |
|---|---|
| Component | Amount (% by weight) |
| Water | 51.05 |
| Disodium EDTA | 0.2 |
| Xanthan gum | 0.5 |
| Cocamidopropyl betaine | 5 |
| Glycerine | 5 |
| Syloid® 244FP hydrated silica | 17 |
| PPG-15 stearyl ether | 10 |
| Polysorbate 60 | 2.5 |
| Hexylene glycol | 6 |
| Menthyl lactate | 0.25 |
| Phenoxetol | 1 |
| Titanium dioxide | 1 |
| Fragrance | 0.3 |
| FD&C Green #3 (0.1% solution) | 0.2 |

The composition formed a pale blue, opaque, homogenous cream.

| Skin Care Composition G) | |
|---|---|
| Component | Amount (% by weight) |
| Water | 52.45 |
| Disodium EDTA | 0.2 |
| Xanthan gum | 0.5 |
| PEG-6 Caprylic/capric glycerides | 1.5 |
| Glycerine | 5 |
| Syloid® 244FP hydrated silica | 17 |
| PPG-15 stearyl ether | 10 |
| Cocamidopropyl betaine | 2 |
| Isoceteth-20 | 2.5 |
| Hexylene glycol | 6 |
| Menthyl lactate | 0.25 |
| Phenoxetol | 1 |
| Titanium dioxide | 1 |
| Fragrance | 0.3 |
| FD&C Green #3 (0.1% solution) | 0.3 |

The composition formed a light green/ blue, opaque cream. The viscosity was 798,400 cps as measured on a Brookfield RVT viscometer using spindle #7 at 5 rpm.

| Skin Care Composition H) | |
|---|---|
| Component | Amount (% by weight) |
| Water | 51.73 |
| Disodium EDTA | 0.2 |
| Xanthan gum | 0.5 |
| PEG-6 Caprylic/capric glycerides | 1.5 |
| Glycerine | 5 |
| Syloid® 244FP hydrated silica | 17 |
| PPG-15 stearyl ether | 10 |
| Cocamidopropyl betaine | 2 |
| Isoceteth-20 | 2.5 |
| Salicylic acid | 0.5 |
| Propylene glycol | 6 |
| Menthyl lactate | 0.25 |
| Phenoxetol | 1 |
| Titanium dioxide | 1 |
| Fragrance | 0.3 |
| FD&C Blue #1 (0.1% solution) | 0.52 |

The composition formed a light blue, opaque cream. The viscosity was 384,000 cps as measured on a Brookfield RVT viscometer using spindle #7 at 5 rpm.

| Skin Care Composition I) | |
|---|---|
| Component | Amount (% by weight) |
| Water | 52.75 |
| Disodium EDTA | 0.2 |
| Xanthan gum | 0.5 |
| PEG-6 Caprylic/capric glycerides | 1.5 |
| Glycerine | 5 |
| Syloid® 244FP hydrated silica | 17 |
| C12-C15 alkyl benzoate | 7.5 |
| Cocamidopropyl betaine | 2 |
| Isoceteth-20 | 2.5 |
| Salicylic acid | 0.5 |
| Propylene glycol | 6 |
| Menthyl lactate | 0.25 |
| Phenoxetol | 1 |
| Titanium dioxide | 1 |
| Dimethicone propyl PG-betaine | 1 |
| Fragrance | 0.3 |
| FD&C Blue #1 (0.1% solution) | 1 |

The composition formed a pale blue, opaque cream. The viscosity was 295,200 cps as measured on a Brookfield RVT viscometer using spindle #7 at 5 rpm.

| Skin Care Composition J) | |
|---|---|
| Component | Amount (% by weight) |
| Water | 54.11 |
| Sodium citrate (20% solution) | 0.14 |
| Xanthan gum | 0.5 |
| PEG-6 Caprylic/capric glycerides | 1.5 |
| Glycerine | 5 |
| Syloid® 244FP hydrated silica | 17 |
| C12-C15 alkyl benzoate | 7.5 |
| Cocamidopropyl betaine | 2 |
| Isoceteth-20 | 2.5 |
| Salicylic acid | 0.5 |
| Propylene glycol | 6 |
| Menthyl lactate | 0.25 |
| Phenoxetol | 0.7 |
| Titanium dioxide | 1 |
| Dimethicone propyl PG-betaine | 1 |
| Fragrance | 0.3 |
| FD&C Blue #1 (0.1% solution) | 0.0008 |

The composition formed a pale blue, opaque cream. The viscosity was 295,200 cps as measured on a Brookfield RVT viscometer using spindle #7 at 5 rpm.

The samples prepared above were split and stored at 4C, 25C, 40C, and 50C for physical stability testing. Stored samples were checked at 4 weeks and 8 weeks. No significant physical stability problems (such as phase separation or precipitate formation) were detected. The samples were also tested for 3 cycles of freeze/ thaw stability. No significant physical stability problems were detected.

### Example 2 - Use Of A Facial Mask Composition

A thin layer of the composition of Example 1 skin care composition A was applied by hand to dry facial skin, avoiding the mouth and eye areas. The mask was light blue in color when applied wet to the face. The color of the mask changed from light blue to white in about 5 minutes, indicating that the mask was dry. The mask was then thoroughly rinsed off with warm tap water. The face was patted dry with a towel. The skin felt clean and not overdry.

A separate test was performed with 100 woman ranging from 13 to 24 years old. The panelists used the composition of Example 1 two to three times over a two week period. The panelists were then asked several questions about the compositions. The results are reported in Table 1 as percent of panelists that agreed.

**Table 1**

| Question | Percent Agreed |
|---|---|
| Removes all traces of dirt and oil. | >90 |
| Cleans deep down. | >90 |
| Extremely easy/very easy to say when product is rinse ready. | 80 |

Based on the data above, the compositions of the present invention are useful as facial cleansing masks that change color upon drying.

## Claims

1. A composition for forming a cosmetic mask comprising:
at least one colorant; and
silica,
wherein, when the composition is combined with water, the amount of the colorant and the silica is effective to provide a color change upon drying.

2. The composition of claim 1 further comprising as a vehicle water, a water miscible liquid or a combination thereof.

3. The composition of claim 2 wherein the vehicle is water and the amount of water ranges from 40 to 90 percent by weight, based on the total weight of the composition.

4. The composition of claim 3 wherein the composition is a paste, a cream, a lotion or a gel.

5. The composition of any one of claims 1 to 4 further comprising at least one nonionic surfactant, anionic surfactant, cationic surfactant, amphoteric surfactant cleansing agent or a mixture thereof.

6. The composition of claim 5 wherein the amount of cleansing agent is from 0.1% to 10% by weight, based upon the total weight of the composition.

7. The composition of any one of claims 1 to 6 further comprising from 0.1 to 30 percent by weight, based on the total weight of the composition, of polypropylene glycol-15 stearyl ether, polypropylene glycol-10 cetyl ether, steareth-10, oleth-8, PPG-4 lauryl ether, C₁₂ - C₁₅ alkyl benzoate, vitamin E acetate, polyethylene glycol-7 glyceryl cocoate, lanolin, glycerine or a combination thereof as an emollient.

8. The composition of any one of claims 1 to 8 further comprising from 0.1 to 40 percent by weight, based on the total weight of the composition of glycerol, a polyalkylene glycol, an alkylene polyol or a derivative, including propylene glycol, dipropylene glycol, polypropylene glycol, polyethylene glycol or a derivative thereof, sorbitol, hydroxypropyl sorbitol, hexylene glycol, 1,3-dibutylene glycol, 1,2,6,-hexanetriol, ethoxylated glycerol, propoxylated glycerol or a mixture thereof as a humectant.

9. The composition of any one of claims 1 to 8 wherein the colorant is FD&C Blue #1 Lake, FD&C Blue #2 Lake, FD&C Red #40 Lake, Erythrosin Lake, Amaranth Lake, Ponceau 4R Lake, Carmoisosine Lake, Carmine Lake, anatto or a colorant generated by converting a naturally derived dye to an aluminum or calcium based salt.

10. The composition of any one of claims 1 to 9 wherein the silica is fumed silica, hydrated silica, a silica gel, precipitated silica or a molecular sieve.

11. The composition of any one of claims 1 to 10 for use in providing a benefit, such as lightening, refreshing, protecting, firming, slimming, cleansing, exfoliating, moisturizing, relieving itching, reducing wrinkles or providing antibacterial protection, to the skin of a human.
